(19) Europäisches Patentamt
European Patent Office
Office européen des brevets

(11) **EP 2 416 769 B1**

(12) **FASCICULE DE BREVET EUROPEEN**

(45) Date de publication et mention
de la délivrance du brevet:
**26.09.2018 Bulletin 2018/39**

(21) Numéro de dépôt: **10723232.4**

(22) Date de dépôt: **09.04.2010**

(51) Int Cl.:
*A61K 31/192* (2006.01)    *B82Y 5/00* (2011.01)
*C07C 65/26* (2006.01)    *A61K 47/69* (2017.01)

(86) Numéro de dépôt international:
**PCT/FR2010/050682**

(87) Numéro de publication internationale:
**WO 2010/116099 (14.10.2010 Gazette 2010/41)**

(54) **PROCEDE DE PREPARATION DE COMPLEXES MOLECULAIRES ENTRE ADAPALENE ET DES CYCLODEXTRINES**

VERFAHREN FÜR DIE HERSTELLUNG MOLEKULARER KOMPLEXE ZWISCHEN ADAPALEN UND CYCLODEXTRINEN

METHOD FOR PREPARING MOLECULAR COMPLEXES BETWEEN ADAPALENE AND CYCLODEXTRINS

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **09.04.2009 FR 0952345**

(43) Date de publication de la demande:
**15.02.2012 Bulletin 2012/07**

(73) Titulaire: **Galderma Research & Development 06410 Biot (FR)**

(72) Inventeurs:
• **LOCHARD, Hubert**
**F-81600 Brens (FR)**
• **FREISS, Bernard**
**F-81100 Castres (FR)**

(74) Mandataire: **Casalonga**
**Casalonga & Partners**
**Bayerstraße 71/73**
**80335 München (DE)**

(56) Documents cités:
**WO-A1-2004/096284    WO-A1-2005/112637**
**WO-A1-2006/070093    WO-A2-2006/042858**

• **PIEL G ET AL:**
**"Betamethasone-in-cyclodextrin-in-liposome : The effect of cyclodextrins on encapsulation efficiency and release kinetics",**
**INTERNATIONAL JOURNAL OF PHARMACEUTICS, ELSEVIER, AMSTERDAM, NL, vol. 312, no. 1-2, 7 April 2006 (2006-04-07), pages 75-82, XP027972695, ISSN: 0378-5173 [retrieved on 2006-04-07]**

EP 2 416 769 B1

**Description**

La présente invention se situe dans le domaine de la chimie et de la pharmacie et concerne un procédé de préparation de complexes moléculaires solubles par la technologie des fluides denses sous pression, notamment celle du CO2. En particulier, la présente invention se rapporte à un procédé de préparation de complexes moléculaires entre Adapalene et des cyclodextrines par la technologie de fluides denses sous pression.

Les nouvelles molécules pharmaceutiques, à forte valeur ajoutée, sont dans 40% des cas insolubles ou peu solubles dans l'eau, ce qui nuit à leur biodisponibilité.

Dans les domaines pharmaceutiques, cosmétique et nutraceutique, il existe un certain nombre de demandes de brevets, brevets et publications relatifs à la formation, en milieu sous pression, de complexes moléculaires d'une substance active dans un substrat enrobant. Néanmoins, la plupart des procédés décrits ne concerne pas l'objectif d'amélioration de la biodisponibilité, mais plutôt l'adsorption d'une substance active sur un substrat.

Van Hees et al. (Application of superchtical carbon dioxide for tAze préparation of a Piroxicam-ss-cyclodextrin inclusion compound, Pharmaceutical Research, Vol. 16, N 12, 1999) décrivent dans leur publication un procédé d'inclusion de Piroxicam dans les (3-cyclodextrines par CO2 supercritique. Le piroxicam étant peu soluble dans l'eau, son inclusion dans les P-cyclodextrines doit permettre d'augmenter sa solubilité dans l'eau. Le procédé consiste à placer un mélange de piroxicam et de (3- cyclodextrines dans un réacteur, laissé en mode statique. Après dépressurisation le mélange obtenu est broyé et homogénéisé avant caractérisation par DSC (Differential Scarming Calorimetry), mesure de solubilité dans l'acétonitrile et comparaison avec la solubilité du piroxicam seul, et méthodes spectroscopiques.

L'analyse par DSC permet de conclure quant à la complexation du piroxicam avec la p-cyclodextine.

Dans la demande WO2006/070093, il est décrit une composition comprenant de l'adapalene solubilisé en milieux aqueux avec des cyclodextrines ou des dérivés de celles-ci. La composition comprend donc un mélange physique entre l'adapalene et les cyclodextrines.

La demande WO2004/096284 décrit un procédé de préparation de complexes moléculaires solubles comprenant une ou plusieurs substances actives peu solubles dans un milieu aqueux incluses dans un ou plusieurs molécules hôtes et en particulier des analgésiques, des antipyrétiques, des antibiotiques, des anti-inflammatoires. Ledit procédé comprend une étape (a) de mise en contact d'une ou plusieurs substances actives avec une ou plusieurs molécules hôtes, une étape (b) de mise en oeuvre d'une étape de diffusion moléculaire par mise en contact, en mode statique, d'un fluide dense sous pression avec le mélange obtenu à l'étape (a) en présence d'un ou plusieurs agents de diffusion, (c) récupération du complexe moléculaire ainsi formé.

De façon surprenante, les inventeurs de la présente demande ont découvert qu'un procédé comprenant une étape de diffusion moléculaire par un fluide dense sous pression en mode statique et dépourvu de l'étape de lavage subséquente à l'aide d'un fluide supercritique, améliorait significativement le taux d'inclusion de la substance active et en particulier en fonction de la quantité d'un agent de diffusion ajoutée au milieu.

En effet, comme démontré dans les exemples, ce procédé permet d'obtenir un complexe moléculaire comprenant de l'Adapalene et des cyclodextrines dont la solubilité est très améliorée par rapport au mélange physique avec de l'Adapalene et des cyclodextrines.

Ainsi, la présente invention concerne un procédé de préparation de complexes moléculaires solubles comprenant au moins de l'Adapalène ou l'un de ses sels inclus dans une méthyl béta cyclodextrine de type « randomised » méthyl β-cyclodextrine ou RAMEB, caractérisé en ce qu'il est constitué par les étapes suivantes :

(a) mise en contact de l'Adapalène ou l'un de ses sels avec ladite méthyl béta cyclodextrine.
(b) mise en oeuvre d'une étape de diffusion moléculaire par mise en contact d'un fluide utilisé à une température ou à une pression supérieure à leur valeur critique avec le mélange obtenu, à l'étape (a) en présence d'un ou plusieurs agents de diffusion choisie(s) parmi les alcools, cette étape étant réalisée en mode statique par mise en présence de tous les réactifs simultanément puis la réaction est laissée se dérouler.
(c) récupération du complexe moléculaire ainsi formée.

Le procédé décrit ci-dessus peut comprendre une étape supplémentaire d) de séchage du complexe, avantageusement à une température comprise entre 40°C et 60°C de préférence sous vide.

La présente invention se rapporte à un procédé de préparation de complexes moléculaires entre l'Adapalene et des cyclodextrines par la technologie de fluides denses sous pression.

Par fluide dense sous pression, on entend au sens de la présente invention tout fluide utilisé à une température ou une pression supérieure à leur valeur critique. Avantageusement il s'agit du pur ou en mélange avec un solvant organique classiquement utilisé par l'homme du métier.

Par sa capacité à lier les récepteurs RAR et/ou RXR, L'adapalène est décrit dans la demande de brevet EP 0 199 636 comme un composé issu de la famille des rétinoïdes benzonaphtaléniques sous le nom d'acide 6-(3-(1-adamantyl)-4-méthoxyphényl)-2-naphtoïque (adapalène).

En particulier, on peut employer l'adapalène ainsi que ses sels. On entend par sels de l'adapalène, les sels formés avec une base pharmaceutiquement acceptable, notamment des bases minérales telles que la soude, la potasse et l'ammoniaque ou des bases organiques telles que la lysine, l'arginine, la N-méthyl-glucamine.

On entend également par sels de l'adapalène les sels formés avec des aminés grasses telles que la dioctylamine et la stéarylamine.

Les concentrations en Adapalène préférées sont comprises entre 0,0001 et 20 % en poids par rapport au poids total de la composition.

De manière préférée, la composition selon l'invention comprend entre 0.001 et 5% et avantageusement entre 0.01 et 1 % en poids d'adapalène par rapport au poids total de la composition, préférentiellement entre 0.01 et 0.5%, de préférence, entre 0.1 et 0.4 % en poids d'adapalène, encore plus préférentiellement 0.3 % en poids d'adapalène.

Les cyclodextrines utilisées dans la présente invention sont celles connues de la littérature.

Les cyclodextrines (CD) sont des oligosaccharides cycliques constitués d'unités ($\alpha$-1,4) $\alpha$-D- glucopyranose avec une cavité centrale lipophile et une surface externe hydrophile (Frómming KH, Szejtli J : « Cyclodextrins in pharmacy », Kluwer Académie Publishers, Dortrecht, 1994).

Les cyclodextrines sont connues pour augmenter la solubilité de molécules par la formation d'une structure en forme de « cage » possédant une partie hydrophile externe et une partie hydrophobe interne. Les cyclodextrines peuvent ainsi former des complexes d'inclusion avec beaucoup de médicaments en acceptant à l'intérieur de la cavité la molécule entière, ou plus communément la partie lipophile de la molécule.

Les cyclodextrines naturelles les plus abondantes sont les $\alpha$-cyclodextrines, les $\beta$-cyclodextrines et les $\gamma$-cyclodextrines. Les $\alpha$-cyclodextrines (connues également sous le nom de Schardinger's $\alpha$- dextrin, cyclomaltohexaose, cyclohexaglucan, cyclohexaamylose, $\alpha$-CD, ACD, C6A) comprennent 6 unités de glucopyranose. Les $\beta$-cyclodextrines (connues également sous le nom Schardinger's $\beta$-dextrin, cyclomaltoheptaose, cycloheptaglucan, cycloheptaamylose, $\beta$-CD, BCD, C7A) comprennent 7 unités de glucopyranose, et les $\gamma$-cyclodextrines (connues également sous le nom Schardinger's $\gamma$-dextrin, cyclomaltooctaose, cyclooctaglucan, cyclooctaamylose, $\gamma$-CD, GCD, C8A) comprennent 8 unités de glucopyranose. Parmi ces trois types de CDs, les $\beta$-cyclodextrines apparaissent comme les agents pharmaceutiques complexants les plus utiles en raison de la taille de leur cavité, de leur disponibilité, de leurs propriétés et de leur faible coût.

Selon Dr J. Szejtli (« Cyclodextrins », in Encyclopedia of Supramolecular Chemistry, eds. Marcel Dekker, 2004) les cyclodextrines sont avantageuses mais présentent également des facteurs limitants qui restreignent l'application des cyclodextrines à certains types de produits pharmaceutiques. Par ailleurs, tous les produits ne sont pas appropriés pour une complexation avec des cyclodextrines. Beaucoup de produits ne peuvent pas être complexés ou bien la complexation ne procure aucun avantage essentiel. Les composés inorganiques sont en général inappropriés à la complexation avec des cyclodextrines.

Dans la présente invention, on utilise la « randomised » méthyl $\beta$-cyclodextrine connue sous le nom de RAMEB.

Le ratio molaire entre l'agent actif et la ou les cyclodextrine(s) sont de l'ordre de 1 :1 à 1 :10. Dans un mode préféré, le ratio molaire est 1 :2 ou 1 :4 ou 1 :6 ou 1 : 8 ou encore 1 :10.

Par agent de diffusion, on entend au sens de la présente invention un solvant favorisant une interaction de la substance active avec la molécule-hôte. Cet agent de diffusion est choisi dans le groupe constitué par les alcool.

Par « mode statique » on entend au sens de la présente invention une réaction ou un procédé dans lequel tous les réactifs sont mis simultanément en présence et où on laisse la réaction se dérouler. Par exemple, dans l'étape (b) de la présente invention, on met dans un autoclave la ou les substance (s) active (s), de un alcool et du C02 supercritique et on laisse réagir pendant plusieurs heures. La masse de produit n'évolue pas durant la réaction. A l'inverse, en mode dynamique, les réactifs sont apportés au fur et à mesure de l'évolution de la réaction ou de la production.

Souvent dans le cadre d'un mode dynamique, il y a circulation d'un fluide ou agitation. La masse de produit évolue durant la production.

L'adapalène et la cyclodextrine sont introduites sous forme solide ou liquide dans un récipient dans lequel est injecté le fluide dense sous pression et l'agent de diffusion dans des proportions judicieusement choisies. Les conditions de pression et de température ainsi que la durée du traitement sont définies, par toute méthode appropriée, en fonction de la nature de la ou des substances actives et du ou des molécules-hôtes.

De façon avantageuse, l'étape (b) de diffusion moléculaire du procédé selon la présente invention est réalisée sous agitation.

Le temps nécessaire à la diffusion moléculaire de l'étape (b) est déterminé par toute méthode appropriée. Cette étape (b) peut être réitérée autant de fois que souhaitée pour obtenir une vitesse de dissolution satisfaisante.

Avantageusement, l'étape (b) dure entre environ 2 et 16 heures.

Les conditions de pression et de température de l'étape (b) sont choisies de façon à favoriser la diffusion moléculaire. Avantageusement la pression du fluide supercritique est comprise entre 5 MPa et 40 MPa et. la température entre 0°C et 120°C. Dans un mode préféré de l'invention, les conditions de température de l'étape b) se situent entre 60°C et 90°C et de manière préférentielle entre 60°C et 85°C.

Avantageusement l'étape (b) du procédé selon la présente invention est mise en oeuvre dans un réacteur fermé en haute pression.

Dans un mode alternatif de la présente invention, le procédé comporte une étape supplémentaire (b') dans la quelle, la ou les cyclodextrine (s) sont ajoutées en excès. Dans un mode préféré, l'excès de cyclodexthne(s) est ajouté pour obtenir en fin de procédé une concentration totale de cyclodextrine(s) de l'ordre de 24 g/l à 98 g/l et de préférence entre 60 g/l et 85 g/l.

Le procédé peut être mis en oeuvre en batch ou comme décrit dans la demande de brevet WO03/043604. De façon avantageuse le procédé selon la présente invention est réalisé en batch.

La mise en oeuvre de l'étape de diffusion moléculaire en milieu dense sous pression en présence d'un agent de diffusion permet une forte interaction des particules de substance active avec la molécule hôte, ce qui favorise la dissolution en milieu aqueux, laquelle est multipliée par environ 100 par le procédé selon la présente invention.

Compte tenu de l'activité marquée de l'Adapalene dans les domaines de la différenciation et de la prolifération cellulaire, les complexes de l'invention conviennent particulièrement bien dans les domaines thérapeutiques suivants :

1) pour traiter les affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle, l'hidradenite supurative,

2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),

3) pour traiter d'autres affections dermatologiques liées à un trouble de la kératinisation avec une composante inflammatoire et/ou immuno- allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation telles que les folliculites,

4) pour traiter toutes les proliférations dermiques ou épidermiques qu'elles soient bénignes ou malignes, qu'elles soient ou non d'origine virale telles que verrues vulgaires, les verrues planes, le molluscum contagiosum, et l'épidermodysplasie verruciforme, les papillomatoses orales ou florides et les proliférations pouvant être induites par les ultraviolets notamment dans le cas des kératoses actiniques,

5) pour réparer ou lutter contre le vieillissement de la peau, qu'il soit photo-induit ou chronologique, ou pour réduire les pigmentations, ou toutes pathologies associées au vieillissement chronologique ou actinique,

6) pour traiter de manière préventive ou curative les troubles de la cicatrisation, les ulcères cutanés, pour prévenir ou pour réparer les vergetures, ou encore pour favoriser la cicatrisation,

7) pour lutter contre les troubles de la fonction sébacée tels que l'hyperséborrhée de l'acné ou la séborrhée simple,

8) dans le traitement de toute affection d'origine fongique au niveau cutané tel que le tinea pedis et le tinea versicolor,

9) dans le traitement d'affections dermatologiques à composante immunologique,

10) dans le traitement de désordres cutanés dus à une exposition aux rayonnements U.V., et

11) dans le traitement d'affections dermatologiques liées à une inflammation ou une infection des tissus environnants le follicule pileux, notamment dues à une colonisation ou infection microbienne notamment l'impétigo, la dermite séborrhéique, la folliculite, le sycosis barbae ou impliquant tout autre agent bactérien ou fongique.

De préférence, les complexes selon l'invention sont particulièrement adaptés au traitement, de manière préventive ou curative, des acnés vulgaires.

Les exemples suivants de mise en oeuvre du procédé sont donnés à titre indicatif non limitatif.

**Exemple 1 : Obtention d'un complexe comprenant de l'Adapalene (substance active) et différents types de cyclodextrines (molécule-hôte)**

Cet exemple a pour but de vérifier la complexation de l'adapalène avec des cyclodextrines par $CO_2$ supercritique afin d'augmenter la solubilité aqueuse du principe actif.

Le rendement de complexation est évalué par mesure de la réduction (ou disparition) du pic thermique relatif au principe actif resté libre.

*Matières utilisées*

Le tableau I ci-dessous répertorie l'ensemble des matières utilisées au cours de cette étude.

|  | M (g/mol) | eau g%g | N° Lot |
|---|---|---|---|
| Adapalène | 412,52 | / | RM000144K1263 |
| Cyclodextrine alpha | 972 | 9 % | 60P304 |
| Cyclodextrine CRISMEB | 1190 | 2,6 % | E001 LAB 3487 |
| Cyclodextrine M-beta N = 7*1,8 | 1311,4 | 1,00 % | 71P018 |
| Eau distillée stérile | 18 | / | MP 7005 |

*Conditions opératoires*

Les conditions opératoires sont fixées par défaut :

- Une mole d'adapalène avec l'équivalent de plusieurs moles de cyclodextrines.
- Ajout d'eau pour atteindre une teneur de 20 % pour les cyclodextrines naturelles, 10 % pour les cyclodextrines greffées.
- Maturation de 2 heures à 60°C et 150 bars
- Séchage à 50°C sous vide durant une nuit.

*Méthodes analytiques*

La teneur en principe actif et les cinétiques de dissolution ont été effectuées en suivant les méthodes décrites ci-après.

**a) Méthode de Dosage**

Conditions chromatographiques HPLC :

- Colonne : Symmetry C18 250x4.6 mm 5 $\mu$m
- Phase mobile : 430 volumes d'acétonitrile
  360 volumes de tétrahydrofurane
  210 d'eau
  0.2 volume d'acide trifloroacétique
- Appareillage HPLC : WATERS 2690 / 2487
- Débit : 1 ml / min
- Longueur d'onde : 270 nm
- Sensibilité du détecteur : 2 AUFS
- Volume injecté : 10 $\mu$l
- Température du four : 25°C
- Temps d'analyse : 15 minutes

**Préparation des solutions :**

*Solutions à examiner :*

Introduire l'équivalent de 200 mg d'Adapalène exactement pesés dans une fiole de 25 ml. Dissoudre et compléter au volume avec du Diméthylformamide (DMF) HPLC. Prélever 1.0 ml de solution dans une fiole de 20 ml. Ajouter 5 ml de DMF, compléter au volume avec la phase mobile.

*Solution témoin :*

SM : Introduire 100 mg d'Adapalène témoin dans une fiole de 100 ml. Dissoudre et compléter au volume avec du Tétrahydrofurane (THF).
Gamme T1 : Dilution au 1/1000ème de SM dans la phase mobile (0.001mg/ml)

T2 : Dilution au 1/100ème de SM dans la phase mobile (0.010 mg/ml)
T3 : Dilution au 1/50ème de SM dans la phase mobile (0.020 mg / ml)
T4 : Dilution au 1/20ème de SM dans la phase mobile (0.050 mg / ml)
T5 : Dilution au 1/10ème de SM dans la phase mobile (0.100 mg / ml)

*Réalisation de l'essai :*

Injecter 10 µl de chaque solution témoin.

Effectuer une régression linéaire des surfaces des pics d'Adapalène par rapport aux concentrations. Le coefficient de corrélation doit être supérieur à 0.995.

Réaliser 2 préparations par essai

Injecter 20 µl des solutions à examiner. Mesurer l'aire du pic d'Adapalène dans chaque solution à examiner. En déduire la concentration X en mg/ml d'après la droite de régression des témoins
La teneur en d'Adapalène exprimée en p.cent (m/m) est donnée par la formule :

$$[\text{Adapalène}] \text{ en p.cent (m/m)} = X \times 500 \times 100 / Pe$$

Pe : prise d'essai en mg de la substance à examiner

**b) Cinétique de dissolution à 3 g/l et à 25°C**

Les conditions chromatographiques et les concentrations des solutions témoin sont les mêmes que celle du dosage.

**Appareillage :**

- Agitation : Banc 15 positions
- Bain thermostaté : 25°C +/- 2°C vérifié sur sonde Prolabo PR 531
- HPLC Waters 2690 - Détecteur 2487/2996
- Pesées : Balance Sartorius A200
- Dilutions : Micro-pipette Eppendorf Research 1000, Eppendorf Research 5000, Gilson M1000
- Station d'eau Ultrapure : ELGA

**Conditions opératoires :**

Dans un erlenmeyer de 100 ml, introduire une prise d'essai équivalente à 150 mg d'Adapalène. Ajouter 50 ml d'eau. Mettre sous agitation magnétique à 400 rpm ou position 4 dans un bain-marie à 25°C +/- 2°C. Effectuer un prélèvement de 2 ml sous agitation magnétique à 15, 30, 60, 120 et 1140 minutes. Filtrer ces prélèvements sur filtres polypropylène 0.45µm Gelman GHP Acrodisc. La solution doit être limpide. Diluer les prélèvements dans la phase mobile d'un facteur « a » permettant d'avoir un pic d'Adapalène de surface comprise entre les surfaces des Témoin 1 et Témoin 5.

*Réalisation de l'essai*

Injecter 10 µl de chaque solution témoin. Effectuer une régression linéaire des surfaces des pics de Adapalène par rapport aux concentrations. Le coefficient de corrélation doit être supérieur à 0.995.

Injecter 10 µl des solutions à examiner.

Mesurer l'aire du pic d'Adapalène dans chaque solution à examiner.

En déduire la concentration X en µg/ml d'après la droite de régression des témoins

Calculer la concentration en µg d'Adapalène solubilisé par ml avec la formule

$$[\text{Adapalène}] \text{ en µg/ml} = X \times a$$

Représenter graphiquement la variation de la quantité dissoute en µg/ml en fonction du temps.

Immédiatement, en fin de dissolution, noter l'aspect et mesurer le pH de la solution.

## RESULTATS ET DEROULEMENT DE L'ETUDE

Afin de vérifier la solubilité apparente de l'adapalène en milieux aqueux, trois essais ont été réalisés avec différentes cyclodextrines et en utilisant l'éthanol comme agent de diffusion:

- la cyclodextrine alpha (6 unités glucose)
- la cyclodextrine méthyl-beta CRISMEB
- la cyclodextrine méthyl-beta RAMEB / Ethanol

Ces échantillons ont été analysés et les résultats sont regroupés dans le tableau II, ci-dessous dans lequel sont indiqués :

- Les différents systèmes « cyclodextrine / ratio molaire »
- La référence de l'échantillon
- La teneur massique en principe actif après maturation
- Les résultats de dissolution aqueuse à 15, 30, 60 et 120 minutes de l'adapalène après maturation **(gras)** et pour un mélange physique correspondant (*italique*).
- Le pH du milieu de dissolution après 120 minutes pour la poudre après maturation **(gras)** et pour le mélange physique correspondant (*italique*).

Tableau II

| | cyclo /ratio | % PA | Sans maturation | | | | | Avec maturation | | | | |
|---|---|---|---|---|---|---|---|---|---|---|---|---|
| | | | 15' | 30' | 60' | 120' | pH | 15' | 30' | 60' | 120' | pH |
| TNT-9A | alpha 1 : 2 | 15,9 | *0* | *0* | *0* | *0* | *6* | **0** | **0** | **0** | **0** | **6** |
| TNT-9B | CRISMEB 1 : 2 | 10,1 | *1* | *1* | *2* | *2* | *5* | **6** | **5** | **5** | **4** | **5** |
| TNT-9C | RAMEB EtOH 1.2.1 | 13,2 | *1* | *2* | *2* | *3* | *5* | **382** | **400** | **376** | **346** | **4** |

Cet exemple montre la complexation de l'adapalene avec au moins 2 types de cyclodextrines et montre un solubilité très améliorée dans la cas de la complexation de l'adapalène avec la cyclodextrine méthyl beta (type RAMEB).

**Exemple 2** : **stabilisation du complexe comprenant de l'Adapalene et la cyclodextrine méthyl beta**

Cet exemple a pour but de montrer la stabilité du complexe obtenu dans l'exemple 1 en faisant varier les paramètres de ratio molaire et de température du procédé en étudiant le profil de dissolution du complexe.

Afin de confirmer l'intérêt d'augmenter la concentration de cyclodextrine et de différencier l'effet des cyclodextrines complexées au cours de la maturation avec l'effet des cycldextrines ajoutées au milieu de dissolution, des complexes à différents ratios ont été réalisés par $CO_2$ supercritique (ratio molaire 1:2 ; 1:4 ; 1:6 ; 1:8 ; 1:10). Par ailleurs, pour cette série d'essai, la température de maturation a été de 85°C au lieu de 60°C. Des cinétiques de dissolution ont été ensuite effectuée à 15 minutes, 7 jours, 30 jours et 48 jours (Tableau III).

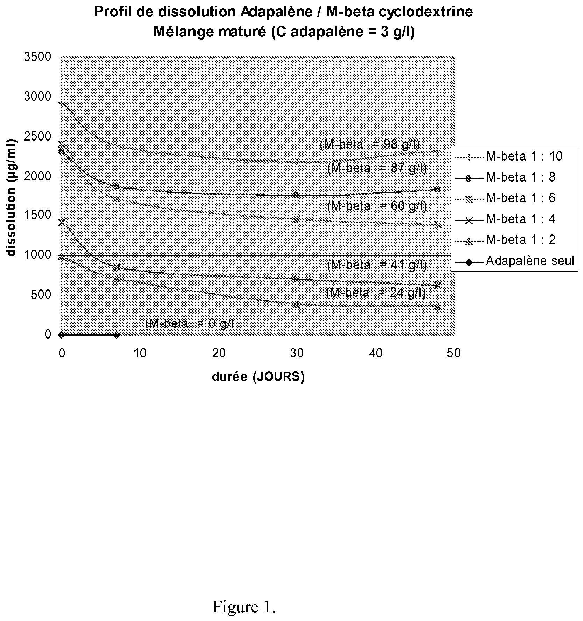

Ces valeurs sont reportées graphiquement sur la figure 1. On constate que l'augmentation de la température de maturation et du ratio de cyclodextrine ont permis d'obtenir une solubilité apparente plus importante même si les profils restent généralement décroissants.

Tableau III

| N° de lot cyclo / ratio | Teneur massique | 15 minutes | 7 jours | 30 jours | 48 jours |
|---|---|---|---|---|---|
| | | **Dissolution** *(pH)* | | | |
| Adapalène seul | 100 % | 0 µg/ml *(5.7)* | 0 µg/ml *(5.7)* | nr | nr |
| TNT-01149 | M-beta / 1 : 2 12,5 % | **993 µg/ml** *(3.9)* | **720 µg/ml** *(3.9)* | **391 µg/ml** *(nr)* | **370 µg/ml** *(4.6)* |
| TNT-02041 | M-beta / 1 : 4 7,3 % | **1413 µg/ml** *(3.9)* | **854 µg/ml** *(3.9)* | **704 µg/ml** *(nr)* | **625 µg/ml** *(4.5)* |
| TNT-02042 | M-beta / 1 : 6 5.0 % | **2406 µg/ml** *(3.8)* | **1723 µg/ml** *(3.8)* | **1456 µg/ml** *(nr)* | **1392 µg/ml** *(4.0)* |
| TNT-02043 | M-beta / 1 : 8 3,4 % | **2307 µg/ml** *(3.7)* | **1867 µg/ml** *(3.8)* | **1755 µg/ml** *(nr)* | **1829 µg/ml** *(3.9)* |
| TNT-02044 | M-beta / 1 : 3,1 % 10 | **2919 µg/ml** *(3.8)* | **2383 µg/ml** *(3.8)* | **2189 µg/ml** *(nr)* | **2318 µg/ml** *(3.8)* |

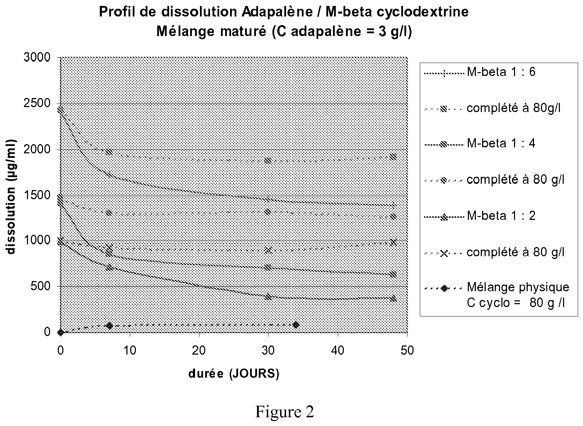

Augmenter le ratio molaire au cours de la maturation revient à augmenter la concentration de cyclodextrine M-beta présent ultérieurement dans le milieu de dissolution. Pour bien dissocier ces deux effets, des profils de dissolution des lots ratio 1:2, 1:4 et 1:6 ont aussi été effectués dans une solution contenant de la cyclodextrine en excès afin de maintenir une concentration totale en cyclodextrine de 80 g/l (la figure 2).

Sur le graphe en figure 2, les courbes pointillés correspondent à des solutions dont les compositions sont strictement identiques (C-adapalène = 3 g/l, C-cyclodextrine = 80 g/l). Les valeurs numériques de ces courbes sont indiquées dans le tableau IV ci-dessous.

Tableau IV

| N° de cyclo / lot ratio | / 15 minutes | 7 jours | 30 jours | 35 jours | 48 jours |
|---|---|---|---|---|---|
| | | **Dissolution** *(pH)* | | | |
| Mélange physique | 2 ng/ml *(5,0)* | 80 µg/ml *(4.5)* | nr | 82 µg/ml *(4.6)* | nr |
| TNT- M-beta / 1 : 01149 2 | **1001 µg/ml** *(4.0)* | **934 µg/ml** *(3.9)* | **896 µg/ml** *(nr)* | nr | **979g/ml** *(4.12)* |
| TNT- M-beta / 1 : 02041 4 | **1468 µg/ml** *(3.9)* | **1304µg/ml** *(3.9)* | **1308 µg/ml** *(nr)* | nr | **1257 µg/ml** *(4.0)* |
| TNT- M-beta / 1 : 02042 6 | **2425 µg/ml** *(3.8)* | **1964 µg/ml** *(3.7)* | **1864 µg/ml** *(nr)* | nr | **1914 µg/ml** *(3.9))* |

Nous pouvons conclure que la cyclodextrine complexée au cours de la maturation contribue à augmenter la solubilité apparente de l'adapalène tandis que la cyclodextrine simplement ajoutée au milieu améliore la stabilité de la solution d'adapalène en maintenant l'état d'équilibre vers la forme complexé.

**Revendications**

1. Procédé de préparation de complexes moléculaires solubles comprenant au moins de l'Adapalène ou l'un de ses sels inclus dans une méthyl béta cyclodextrine de type « randomised » méthyl β-cyclodextrine ou RAMEB, **caractérisé en ce qu'**il est constitué par les étapes suivantes :

(a) mise en contact de l'Adapalène ou l'un de ses sels avec ladite méthyl béta cyclodextrine,

(b) mise en oeuvre d'une étape de diffusion moléculaire par mise en contact d'un fluide utilisé à une température ou à une pression supérieure à leur valeur critique avec le mélange obtenu à l'étape (a) en présence d'un ou plusieurs agents de diffusion choisi(s) parmi les alcools, cette étape étant réalisée en mode statique par mise en présence de tous les réactifs simultanément puis la réaction est laissée se dérouler,

(c) récupération du complexe moléculaire ainsi formé.

2. Procédé selon la revendication 1, **caractérisé en ce qu'**il comprend une étape supplémentaire (d) de séchage du complexe.

3. Procédé selon la revendication 2, **caractérisé en ce que** l'étape (d) est effectuée à une température comprise entre 40°C et 60°C.

4. Procédé selon l'une des revendications 2 ou 3, **caractérisé en ce que** l'étape (d) est effectuée sous vide.

5. Procédé selon la revendication 1, **caractérisé en ce que** le fluide utilisé à une température et une pression supérieure à leur valeur critique est du CO2.

6. Procédé selon l'une des revendications 1 à 5, **caractérisé en ce que** l'étape (b) de diffusion moléculaire est réalisée sous agitation.

7. Procédé selon l'une quelconque des revendications 1 à 5, **caractérisé en ce qu'**il comporte une étape (b') dans laquelle la méthyl béta cyclodextrine est ajoutée en excès.

8. Procédé selon l'une quelconque des revendications 1 à 7, **caractérisé en ce que** le ratio molaire Adapalène/méthyl béta cyclodextrine est compris entre 1/1 et 1/10.

9. Procédé selon la revendication 8, **caractérisé en ce que** le ratio molaire Adapalène/méthyl béta cyclodextrine est compris entre 1/1 et 1/6.

10. Procédé selon la revendication 9, **caractérisé en ce que** le ratio molaire Adapalène/méthyl béta cyclodextrine est compris entre 1/2 et 1/4.

11. Procédé selon l'une quelconque des revendications 1 à 10, **caractérisé en ce que** la pression du fluide supercritique est comprise entre 5 MPa et 40 MPa et la température entre 0° et 120°C.

12. Procédé selon la revendication 11, **caractérisé en ce que** la température du fluide supercritique est comprise entre 60° et 90°C.

**Patentansprüche**

1. Verfahren zur Herstellung löslicher molekularer Komplexe, umfassend mindestens Adapalen oder eines seiner Salze, die in einem Methyl-beta-cyclodextrin vom Typ "randomisiertes" Methyl-$\beta$-cyclodextrin oder RAMEB eingeschlossen sind, **dadurch gekennzeichnet, dass** dieses aus den folgenden Schritten besteht:

(a) Inkontaktbringen von Adapalen oder einem seiner Salze mit Methyl-$\beta$-cyclodextrin,

(b) Durchführen eines Schritts einer molekularen Diffusion durch Inkontaktbringen eines Fluids, das bei einer Temperatur oder einem Druck über ihrem kritischen Wert verwendet wird, mit der in Schritt (a) erhaltenen Mischung in Anwesenheit eines oder mehrerer Diffusionsmittel, die aus Alkoholen ausgewählt werden, wobei dieser Schritt im statischen Modus durch Inberührungbringen aller Reagenzien gleichzeitig durchgeführt wird, wobei die Reaktion anschließend ablaufen gelassen wird,

(c) Gewinnen des so gebildeten molekularen Komplexes.

2. Verfahren nach Anspruch 1, **dadurch gekennzeichnet, dass** dieses einen ergänzenden Schritt (d) des Trocknens des Komplexes umfasst.

3. Verfahren nach Anspruch 2, **dadurch gekennzeichnet, dass** der Schritt (d) bei einer Temperatur zwischen 40 °C und 60 °C durchgeführt wird.

4. Verfahren nach einem der Ansprüche 2 oder 3, **dadurch gekennzeichnet, dass** der Schritt (d) im Vakuum durchgeführt wird.

5. Verfahren nach Anspruch 1, **dadurch gekennzeichnet, dass** das Fluid, das bei einer Temperatur oder einem Druck über ihrem kritischen Wert verwendet wird, $CO_2$ ist.

6. Verfahren nach einem der Ansprüche 1 bis 5, **dadurch gekennzeichnet, dass** der Schritt (b) der molekularen Diffusion unter Rühren durchgeführt wird.

7. Verfahren nach einem der Ansprüche 1 bis 5, **dadurch gekennzeichnet, dass** dieses einen Schritt (b') umfasst, in dem das Methyl-beta-cyclodextrin im Überschuss zugesetzt wird.

8. Verfahren nach einem der Ansprüche 1 bis 7, **dadurch gekennzeichnet, dass** das Molverhältnis Adapalen/Methyl-beta-cyclodextrin zwischen 1/1 und 1/10 liegt.

9. Verfahren nach Anspruch 8, **dadurch gekennzeichnet, dass** das Molverhältnis Adapalen/Methyl-beta-cyclodextrin zwischen 1/1 und 1/6 liegt.

10. Verfahren nach Anspruch 9, **dadurch gekennzeichnet, dass** das Molverhältnis Adapalen/Methyl-beta-cyclodextrin zwischen 1/2 und 1/4 liegt.

11. Verfahren nach einem der Ansprüche 1 bis 10, **dadurch gekennzeichnet, dass** der Druck des superkritischen Fluids zwischen 5 MPa und 40 MPa und die Temperatur zwischen 0 °C und 120 °C liegt.

12. Verfahren nach Anspruch 11, **dadurch gekennzeichnet, dass** die Temperatur des superkritischen Fluids zwischen 60 °C und 90 °C liegt.

**Claims**

1. Process for the preparation of soluble molecular complexes comprising at least adapalene or one of its salts included in a methyl-β-cyclodextrin of "randomized" methyl-β-cyclodextrin or RAMEB type, **characterized in that** it consists in the following stages:

   (a) bringing the adapalene or one of its salts into contact with the said methyl-β-cyclodextrin,
   (b) carrying out a stage of molecular diffusion by bringing a fluid, used at a temperature or at a pressure greater than their critical value, into contact with the mixture obtained in stage (a) in the presence of one or more diffusion agents chosen from alcohols, this stage being carried out in static mode by bringing together all the reactants simultaneously, and then the reaction is allowed to proceed,
   (c) recovering the molecular complex thus formed.

2. Process according to Claim 1, **characterized in that** it comprises an additional stage (d) of drying the complex.

3. Process according to Claim 2, **characterized in that** the stage (d) is carried out at a temperature of between 40°C and 60°C.

4. Process according to either of Claims 2 and 3, **characterized in that** stage (d) is carried out under vacuum.

5. Process according to Claim 1, **characterized in that** the fluid used at a temperature and a pressure greater than their critical value is $CO_2$.

6. Process according to one of Claims 1 to 5, **characterized in that** stage (b) of molecular diffusion is carried out with stirring.

7. Process according to any one of Claims 1 to 5, **characterized in that** it comprises a stage (b') in which the methyl-β-cyclodextrin is added in excess.

8. Process according to any one of Claims 1 to 7, **characterized in that** the adapalene/methyl-β-cyclodextrin molar ratio is between 1/1 and 1/10.

9. Process according to Claim 8, **characterized in that** the adapalene/methyl-β-cyclodextrin molar ratio is between 1/1 and 1/6.

10. Process according to Claim 9, **characterized in that** the adapalene/methyl-β-cyclodextrin molar ratio is between 1/2 and 1/4.

11. Process according to any one of Claims 1 to 10, **characterized in that** the pressure of the supercritical fluid is between 5 MPa and 40 MPa and the temperature between 0°C and 120°C.

12. Process according to Claim 11, **characterized in that** the temperature of the supercritical fluid is between 60°C and 90°C.

**Profil de dissolution Adapalène / M-beta cyclodextrine**
**Mélange maturé (C adapalène = 3 g/l)**

Figure 1.

Figure 2

## RÉFÉRENCES CITÉES DANS LA DESCRIPTION

**Documents brevets cités dans la description**

- WO 2006070093 A
- WO 2004096284 A
- EP 0199636 A
- WO 03043604 A

**Littérature non-brevet citée dans la description**

- **VAN HEES et al.** Application of superchtical carbon dioxide for tAze préparation of a Piroxicam-ss-cyclodextrin inclusion compound. *Pharmaceutical Research,* 1999, vol. 16 (12
- **FRÓMMING KH ; SZEJTLI J.** Cyclodextrins in pharmacy. Kluwer Académie Publishers, 1994
- **DR J. SZEJTLI.** Cyclodextrins. Marcel Dekker, 2004